# EUROPÄISCHE PATENTSCHRIFT

(11) **EP 0 640 393 B1**
(45) Veröffentlichungstag und Bekanntmachung des Hinweises auf die Patenterteilung: **26.05.1999**
(21) Anmeldenummer: 94113122.9
(22) Anmeldetag: 23.08.1994
(51) Int. Cl.: B01L 11/00, B65D 43/26

(54) **System zur Bevorratung von Testelementen**
System for storing test elements
Dispositif pour l'approvisionnement d'éléments de test

(30) Priorität: 27.08.1993 DE 4328815
(43) Veröffentlichungstag der Anmeldung: 01.03.1995
(73) Patentinhaber: Roche Diagnostics GmbH, 68298 Mannheim (DE)
(72) Erfinder: Eikmeier, Heino, Dr., D-64653 Lorsch (DE); Sacherer, Klaus-Dieter, D-67281 Kirchheim (DE)

(56) Entgegenhaltungen:
- DE-A- 3 519 296
- US-A- 1 458 166
- US-A- 2 562 580
- DATABASE WPI Section Ch, Week 8711, Derwent Publications Ltd., London, GB; Class J04, AN 87-077006/11 & JP-A-62 030 962 (FUJI PHOTO FILM KK) 9. Februar 1987

## Beschreibung

Die Erfindung betrifft ein System zur Bevorratung von Testelementen zur Analyse von Probenflüssigkeiten, bei dem sich zwei oder mehr Testelemente in einem Vorratsbehältnis befinden, das wasserdampfdicht verschlossen ist und in seinem Inneren ein Trockenmittel enthalten kann.

Die Erfindung liegt im Bereich der Verpackung und Bevorratung von Testelementen, die in der Regel empfindlich gegenüber Luftfeuchte bzw. Wasserdampf sind.

Im Stand der Technik sind Vorratsbehältnisse für analytische Testelemente bekannt, die zwar für eine wasserdampfdichte Verpackung sorgen, die jedoch Nachteile in ihrer Handhabung aufweisen. Es sind beispielsweise Röhren aus Aluminium bekannt, die mit einem Plastikstopfen verschlossen werden, der auf der Seite, die dem Innenraum der Röhre zugewandt ist, ein Trockenmittel trägt. Der Stopfen dichtet jedoch nur ausreichend, wenn er fest auf dem Röhrenrand aufsitzt. Gut dichtende Stopfen sind daher schwergängig und somit für den Anwender unbequem. Außerdem wird die Röhre häufig für die Zeit der Probenaufgabe offen gelassen, was dazu führt, daß Feuchte an die Testelemente gelangt und das Trockenmittel im Stopfen mit Wasser beladen wird.

In DE-A-35 19296 wird ein Verschluß für Behältnisse von Testelementen beschrieben, der ein Schnappscharnier besitzt. Zum Öffnen bzw. Schließen des Gefäßes wird der Schnappverschluß über einen mechanischen Widerstand hinwegbewegt. Damit der Schnapperschluß im geschlossenen Zustand ausreichend dichtet, um ein Eindringen von Feuchte zu verhindern, muß der Anwender den Schnappverschluß kräftig auf die Öffnung drücken, bis ein Einrasten erfolgt. Ebenso wie im Falle der Aluminiumröhren muß der Anwender selbst dafür Sorge tragen, daß das Gefäß direkt nach der Entnahme von Testelementen ausreichend dicht verschlossen wird.

In der US-1, 458,166 ist ein Vorratsbehältnis für photographische Papiere beschrieben, das eine mit einer Federkraft belegte Klappe besitzt. Die Klappe wird vom Benutzer zur Entnahme eines Papiers geöffnet und verschließt sich nach der Entnahme durch Federkraft selbsttätig.

Es lag der Erfindung daher die Aufgabe zugrunde, ein Aufbewahrungssystem für Testelemente zur Verfügung zu stellen, das einen ausreichenden Schutz der Testelemente vor Luftfeuchte gewährleistet und dem Anwender eine einfache und bequeme Handhabung ermöglicht.

Diese Aufgabe wurde erfindungsgemäß durch eine System gemäß Anspruch 1 gelöst.

Ein erfindungsgemäßes System dient zur Aufbewahrung von Testelementen zur Durchführung von Analysen ohne Verwendung von Reagenzflüssigkeiten. In der Regel besitzen genannte Testelemente die Form eines Streifens, was zu einem System in Art einer länglichen Schachtel führt. Aufgrund der noch näher auszuführenden mechanischen Vorrichtungen sind Schachteln mit rechteckigen Seitenflächen bevorzugt.

Ein erfindungsgemäßes System beinhaltet einen Grundkörper und eine Klappe, wobei der Grundkörper seinerseits ein Vorratsbehältnis beinhaltet, das sich in einer Halterung befindet.

Um dem Anwender die Entnahme von Testelementen zu ermöglichen besitzt ein erfindungsgemäßes System ein Vorratsbehältnis, in dem die Testelemente wie in einem Köcher stecken, aus dem sie bei geöffnetem System entnommen werden können.

Der Grundkörper des Systems besitzt eine Öffnung, welche von einer Klappe verschlossen wird, die durch einen Federmechanismus auf den Grundkörper gedrückt oder gezogen wird. Grundkörper und Klappe besitzen daher einen Satz von Flächen, die bei geschlossenem System dichtend aufeinander liegen. Bevorzugt drückt die Klappe auf die Öffnung des Vorratsbehältnisses und dichtet den Innenraum des Vorratsbehältnisses gegen das Eindringen von Feuchte ab.

Ein Verschluß des Innenraumes des Systems durch die Kombination aus Grundkörper und Klappe wird dadurch verbessert, daß leicht deformierbares Material, wie beispielsweise Moosgummi oder ein weiches Plastik, in den Öffnungsrand des Grundkörpers oder der Klappe eingelegt ist. Bei geschlossenem System dichtet das deformierbare Material den Innenraum des Systems gegen das Eindringen von Wasserdampf ab.

Die Klappe wird von einer Feder, einem Gummiband oder einer anderen Rückholvorrichtungen auf die Öffnung des Grundkörpers gedrückt oder gezogen. Bei geschlossenem System ist die Zugrichtung der Rückholvorrichtung bevorzugt senkrecht zum Öffnungsrand des Grundkörpers, auf der die Klappe aufliegt. Der Federmechanismus der Rückholvorrichtung ist sowohl bei geöffnetem als auch bei geschlossenem System gespannt.

Das System kann vom Benutzer dadurch geöffnet werden, daß er die Klappe gegen den Zug der Rückholvorrichtung vom Grundkörper abzieht. Ein geöffnetes System besitzt keinen Ruhepunkt. Ein Ruhepunkt wird erst erreicht, wenn sich das System selbsttätig verschlossen hat. Durch diese erfindungsgemäße Eigenschaft des Systems wird ausgeschlossen, daß das System ohne Einwirkung des Benutzers geöffnet bleiben kann und die Testelemente für längere Zeit mit der Umgebungsluft in Berührung kommen.

Der Schutz der im System enthaltenen Testelemente kann noch durch Trockenmittel im Vorratsbehältnis erhöht werden. Als Trockenmittel können feste Trockenmittel verwendet werden. Bevorzugt sind Kieselgele und Molekularsiebe.

Die Klappe, die das System verschließt wird bevorzugt senkrecht zur Öffnung des Grundkörpers abgezogen und darauffolgend zur Seite weggeklappt, damit die Öffnung des Grundkörpers zur besseren Entnahme von Testelementen freigegeben wird. Besonders bevorzugt wird vom Benutzer eine Vorrichtung, z. B. ein Schieber oder Hebel, betätigt, der die Klappe öffnet. Dies kann dadurch erfolgen, daß die Klappe gegen den Befestigungspunkt der Rückholvorrichtung verschoben wird oder dadurch, daß die Vorrichtung die Verbindungsstelle der Rückholvorrichtung mit dem Grundkörper verschiebt. In letzterem Falle wird die Zugspannung der Rückholvorrichtung vermindert, worauf sich die Klappe von der Öffnung des Grundkörpers löst.

Ein erfindungsgemäßes System ist als Nachfüllverpackung konzipiert, d. h. das gesamte System wird nach Entnahme der Testelemente nachgefüllt. Bei einem erfindungsgemäß nachfüllbaren System, übernimmt der Grundkörper die Funktion einer Halterung für Nachfüllpackungen mit Testelementen. Nachfüllpackungen werden bevorzugt von der der Klappe gegenüberliegenden Seite in den Grundkörper eingeschoben, es ist erfindungsgemäß jedoch auch möglich, daß das System wie zur Entnahme von Testelementen geöffnet und die Nachfüllpackung durch die freigelegte Öffnung in den Grundkörper hineingeschoben wird.

Die verschlossene Nachfüllpackung ihrerseits muß vor Verwendung im erfindungsgemäßen System eine wasserdampfdichte Verpackung der Testelemente gewährleisten, was jedoch technisch unproblematisch ist, da ein Einmalverschluß verwendet werden kann. Die Anwesenheit von Trockenmittel in der Nachfüllpackung erhöht sowohl die Haltbarkeit der Testelemente bei Lagerung der Nachfüllpackung als auch die Haltbarkeit bei Verwendung in erfindungsgemäßen Systemen.

Die Entsiegelung und Öffnung einer Nachfüllpackung kann dazu z. B. durch das Abziehen einer Banderole erfolgen. Es kann auch eine Entsiegelung beim Einschieben in den Grundkörper des Systems, z. B. durch das Durchstechen einer Versiegelung erfolgen.

Materialien, die zum Aufbau eines erfindungsgemäßen Systems dienen können, werden durch Anforderungen an mechanische Stabilität, gute Bearbeitbarkeit und geringe Wasserdampfdurchlässigkeit, ausgewählt. Für Grundkörper und Klappe kommen in erster Linie Metalle und Kunststoffe in Frage. Während Metalle, wie z. B. Stahl und bevorzugt Aluminium, von vorneherein eine geringe Wasserdampfdurchlässigkeit aufweisen, wird diese Anforderung bei Kunststoffen in der Regel erst mit Materialstärken über 1 mm erreicht. Bevorzugte Kunststoffe sind Polyethylen und Polypropylen. Zur Abdichtung der Auflagefläche von Klappe und Grundkörper werden bevorzugt Plaste, z. B. Moosgummi, eingesetzt.

Genannte Rückholvorrichtungen können beispielsweise Metall- oder Plastikfedern sein oder durch dehnbare Bänder realisiert werden.

Ein erfindungsgemäßes System bietet dem Benutzer den Vorteil, daß es einfach und bequem zu handhaben ist und eine Aufbewahrung von Testelementen unter Abschluß von Luftfeuchte ermöglicht. Ein unvollständiges Verschließen wird durch den Rückholmechanismus vermieden, so daß auch bei Entnahme von einzelnen Testelementen eine lange Haltbarkeit der im System verbleibenden Testelemente gewährleistet wird.

Auf dem System, d. h. auf dem Grundkörper bzw. Halter, dem Vorratsbehältnis oder der Klappe können chargenspezifische Daten der Testelemente alphanummerisch, als Strichcode oder als Magnetstreifen angebracht sein. Dies dient dem Benutzer als Information über den Inhalt des Bevorratungssystems. Gegebenenfalls können diese Daten über ein geeignetes Lesegerät einem Analysengerät mitgeteilt werden.

Eine besonders bevorzugte Ausführungsform eines erfindungsgemäßen Systems wird anhand der folgenden Figuren dargestellt:
- Figur 1:: geschlossenes System
- Figur 2:: geöffnetes System

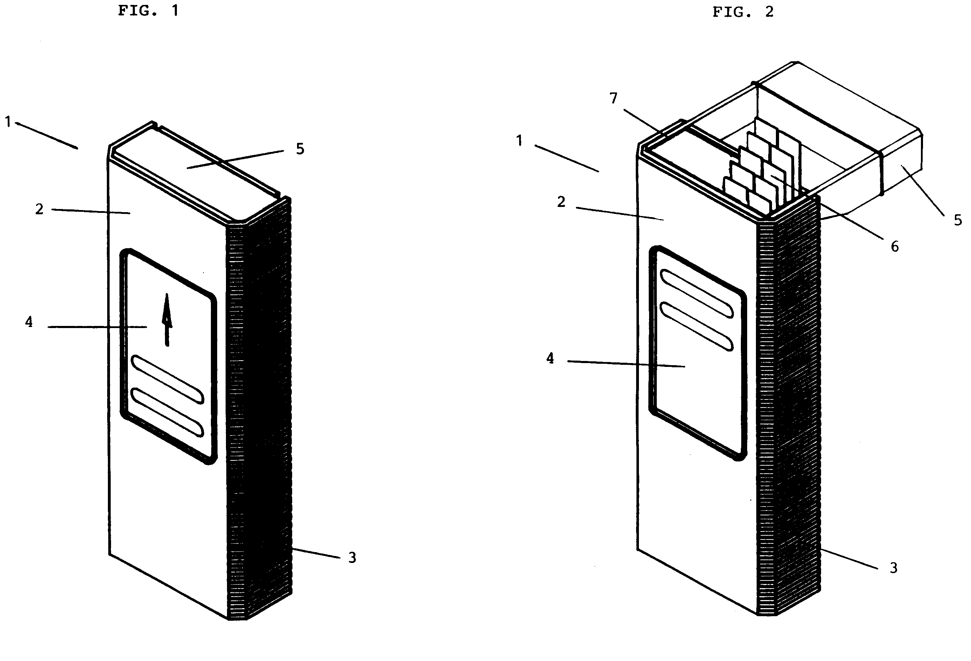

Figur 1 zeigt ein geschlossenes System (1), das die Form einer länglichen Schachtel besitzt. In dieser Ausführungsform besteht der Grundkörper aus einem Halter (2) und einem Vorratsbehältnis (20). Der Halter (2) besitzt an zwei gegenüberliegenden Seiten Riffelungen (3), die als Grifflächen dienen. Auf der Vorderseite befindet sich ein Schieber (4), mit dem die Klappe (5) geöffnet werden kann. Im geschlossenen Zustand schließt die Klappe (5) mit der Oberflächenkontur des Grundkörpers ab.

In Figur 2 ist das System aus Figur 1 dargestellt, nachdem der Schieber (4) nach oben, d. h. in Richtung auf die Klappe (5) verschoben wurde. Die Klappe (5) ist aus dem Halter (2) herausgeschoben und zur Seite weggeklappt, so daß die Teststreifen (6) freiliegen. Um das System, wie in Figur 2 dargestellt, offen zu halten, muß der Schieber (4) in der gezeigten Position festgehalten werden. Wird er losgelassen, so bewegt er sich in die in Figur 1 dargestellte Position zurück und das System verschließt sich automatisch. Eine Abdichtung gegen Wasserdampf aus der Umgebungsluft findet statt, indem die Klappe (5) in geschlossenem Zustand auf das Moosgummi (7), das sich an der Öffnung des Grundkörpers befindet, drückt.

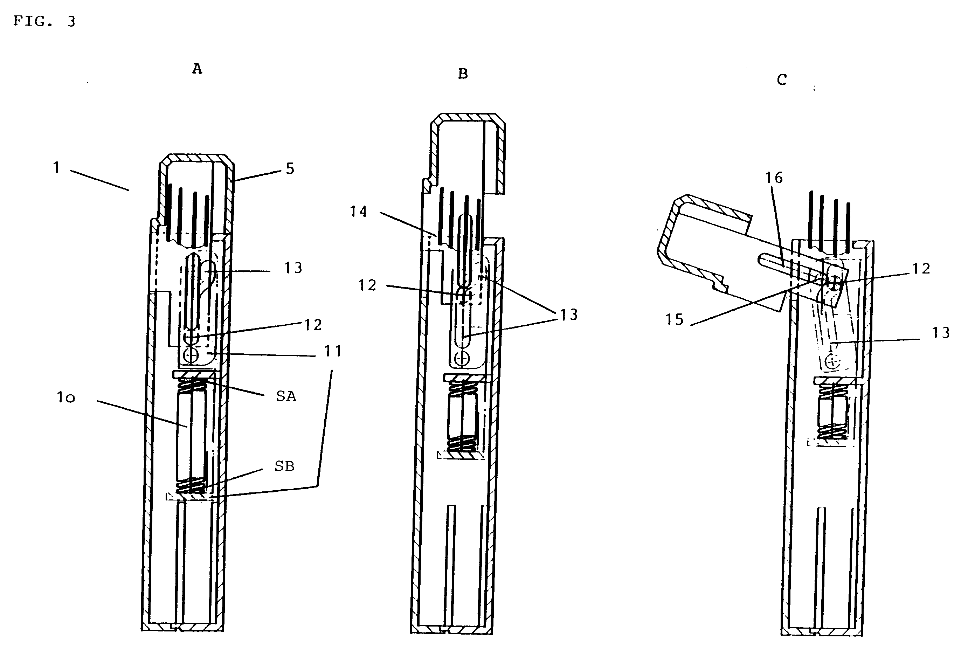

Die Funktionsweise des Öffnungsmechanismus wird durch Figur 3 detaillierter dargestellt. Figur 3A zeigt ein geschlossenes System (1). Die Feder (10) ist an einer ersten Seite (SA) mit dem Halter (2) und an einer zweiten Seite (SB) mit einem Hebel (11) verbunden. Der Hebel (11) bewegt eine Walze (12), die in einer Führungsschiene (13) läuft. Die Walze (12) ist mit der Klappe (5) mechanisch verbunden. Bei Betätigung eines Schiebers wird die Feder (10), wie in Figur 3B dargestellt, zusammengedrückt und die Walze (12) vom unteren Ende der Führungsschiene (13) bis zu einer mittleren Position bewegt. Die Klappe (5) wird durch diese Betätigung von der Öffnung (14) des Systems abgehoben. Figur 3C zeigt, welche Bewegung die Klappe (5) ausführt, wenn die Feder (10) weiter zusammengedrückt wird. Die Walze (12) bewegt sich zum oberen Ende der Führungsschiene (13) und die Klappe (5) kippt um eine Achse, die durch eine zweite Walze (15) gegeben ist. Die zweite Walze (15) ist mit der Führungsschiene (13) mechanisch verbunden und bewegt sich in einer zweiten Führungsschiene (16), die sich in der Klappe (5) befindet.

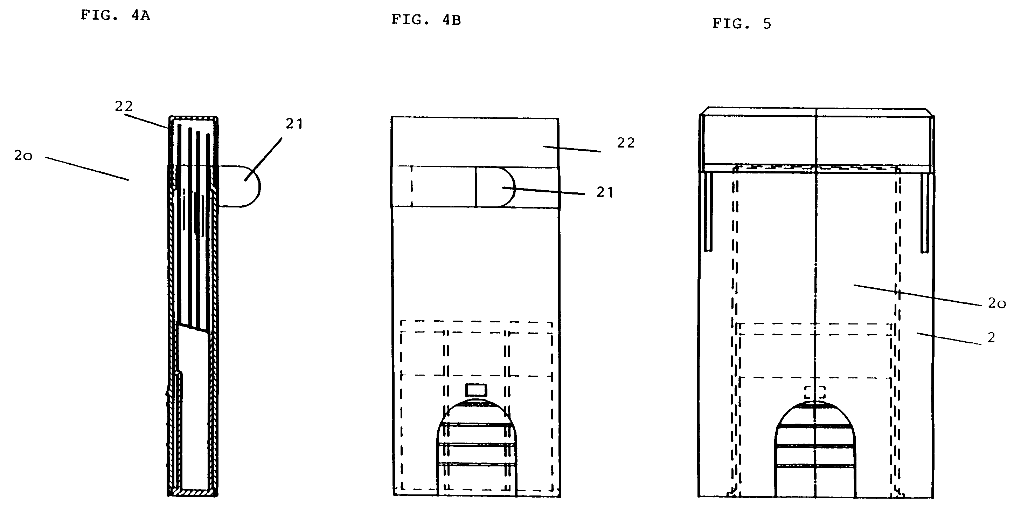

Figuren 4A und 4B zeigen ein Vorratsbehältnis (20) in Seitenansicht. Das Vorratsbehältnis (20) ist in diesem Fall eine versiegelte Nachfüllpackung. Die Nachfüllpackung besitzt eine Aufreißlasche (21), die abgezogen wird, wonach der Verschluß (22) der Nachfüllpackung abgenommen werden kann.

Figur 5 zeigt, wie sich das Vorratsbehältnis (20) innerhalb des Grundkörpers (2) befindet.

### Bezugszeichenliste:

- (1):: System
- (2):: Halter
- (3):: Riffelungen
- (4):: Schieber
- (5): Klappe
- (6):: Teststreifen
- (7):: Moosgummi
- (10):: Feder
- (11):: Hebel
- (12):: Walze
- (13):: Führungsschiene
- (14):: Öffnung
- (15):: zweite Walze
- (16):: zweite Führungsschiene
- (20):: Vorratsbehältnis
- (21):: Aufreißlasche
- (22):: Verschluß
- (SA):: erste Seite
- (SB):: zweite Seite

## Patentansprüche

1. System (1) zur Bevorratung von Testelementen zur Analyse von Probeflüssigkeiten, bei dem sich zwei oder mehr Testelemente in dem System befinden, mit einer Klappe (5) und einen Grundkörper mit Öffnung, dadurch gekennzeichnet, daß
der Grundkörper ein Halter (2) für ein Vorratsbehältnis (20) von Testelementen ist und die Klappe (5) durch eine Rückholvorrichtung mit einem Federmechanismus, der sowohl bei geöffnetem als auch geschlossenem System gespannt ist, selbsttätig und dichtend auf die Öffnung des Grundkörpers oder des Vorratsbehältnisses (20) gedrückt wird, so daß die Testelemente im Inneren des Systems vor dem Zutritt von Wasserdampf geschützt sind.

2. System gemäß Anspruch 1, bei dem sich im Inneren des Vorratsbehältnisses (20) oder des Grundkörpers ein Trockenmittel befindet.

3. System gemäß Anspruch 1, das einen Schieber (4) besitzt, der die Klappe (5) des Systems öffnet.

4. System gemäß Anspruch 1, bei dem das Vorratsbehältnis (20) aus der Halterung entnommen werden kann.

5. System gemäß Anspruch 1, bei dem das Vorratsbehältnis (20) von der der Klappe (5) abgewandten Seite in den Halter (2) eingeführt wird.

6. System gemäß Anspruch 1, bei dem sich an der Klappe (5) oder dem Grundkörper eine Dichtung (7) befindet.

7. System gemäß Anspruch 1, bei dem sich chargenspezifische Daten der Testelemente alphanummerisch, als Strichcode oder als Magnetstreifen, auf dem System befinden.

8. System gemäß Anspruch 1, bei dem das Vorratsbehältnis (20) eine Nachfüllpackung ist.

## Claims

1. System (1) for the storage of test elements for the analysis of sample liquids in which two or more test elements are present in the system with a flap (5) and a basic body with an opening, wherein
the basic body is a holder (2) for a storage container (20) for test elements and the flap (5) is pressed automatically by a return device with a spring mechanism which is tensioned when the system is opened as well as closed, onto the opening of the basic body or of the storage container (20) to form a seal so that the test elements inside the system are protected against entry of water vapour.

2. System as claimed in claim 1, in which a desiccant is present inside the storage container (20) or inside the basic body.

3. System as claimed in claim 1 which has a slide (4) which opens the flap (5) of the system.

4. System as claimed in claim 1, in which the storage container (20) can be removed from the holder.

5. System as claimed in claim 1, in which the storage container (20) can be inserted into the holder (2) from the side facing away from the flap (5).

6. System as claimed in claim 1, in which a seal (7) is present on the flap (5) or the basic body.

7. System as claimed in claim 1, in which batch-specific data of the test elements are located on the system alphanumerically, as bar codes or as magnetic strips.

8. System as claimed in claim 1, in which the storage container (20) is a refill pack.

## Revendications

1. Système (1) pour le stockage d'éléments de test pour analyser des prélèvements liquides, dans lequel se trouvent deux ou plusieurs éléments de test, avec un clapet (5) et un corps de base avec ouverture, caractérisé en ce que le corps de base est un support (2) pour un récipient de stockage (20) d'éléments de test, et le clapet (5) appuie de manière automatique et étanche sur l'ouverture du corps de base ou du récipient de stockage (20), à l'aide d'un dispositif de rappel avec un mécanisme à ressort qui est tendu aussi bien lorsque le système est ouvert que lorsqu'il est fermé, de sorte que les éléments de test à l'intérieur du système sont protégés contre l'entrée de vapeur d'eau.

2. Système selon la revendication 1, dans lequel un agent siccatif se trouve à l'intérieur du récipient de stockage (20) ou du corps de base.

3. Système selon la revendication 1, qui possède un coulisseau de verrouillage (4) qui ouvre le clapet (5) du système.

4. Système selon la revendication 1, dans lequel le récipient de stockage (20) peut être retiré du support.

5. Système selon la revendication 1, dans lequel le récipient de stockage (20) est introduit dans le support (2) par le côté détourné du clapet (5).

6. Système selon la revendication 1, dans lequel un joint d'étanchéité (7) se trouve au niveau du clapet (5) ou du corps de base.

7. Système selon la revendication 1, dans lequel se trouvent des données spécifiques à la charge des éléments de test sous forme alphanumérique, tels que sous forme de code barres ou de bande magnétique.

8. Système selon la revendication 1, dans lequel le récipient de stockage (20) constitue une recharge.
